Europäisches Patentamt

European Patent Office

Office européen des brevets

(19)

(11) Publication number: **0 162 580**
**B1**

(12)

# EUROPEAN PATENT SPECIFICATION

(45) Date of publication of patent specification: **27.07.88**

(21) Application number: **85302751.4**

(22) Date of filing: **18.04.85**

(51) Int. Cl.⁴: **C 13 F 1/02, C 12 G 1/06**

(54) Massecuite supersaturation monitor.

(30) Priority: **19.04.84 ZA 842976**

(43) Date of publication of application:
**27.11.85 Bulletin 85/48**

(45) Publication of the grant of the patent:
**27.07.88 Bulletin 88/30**

(84) Designated Contracting States:
**BE DE FR GB NL**

(56) References cited:
**DE-A-1 567 298**
**FR-A-2 427 391**
**INTERNATIONAL SUGAR JOURNAL, vol. 86, no. 1023, March 1984, pages 73-79, London, GB; G.R. MOLLER: "Recent developments in pan boiling automatics"**
**REVIEW OF SCIENTIFIC INSTRUMENTS, vol. 49, no. 7, 1978, pages 936-939, American Institute of Physics, New York, US; I. OGAWA et al.: "Improved circuit for impedance measurement at very high frequency and its application in testing dielectric properties of insulating materials"**

**The file contains technical information submitted after the application was filed and not included in this specification**

(73) Proprietor: **The Tongaat-Hulett Group Limited Mount Edgecombe Natal (ZA)**

(72) Inventor: **Radford, David John Dudley Pringle Village Maidstone Natal (ZA)**

(74) Representative: **Evershed, Michael et al MARKS & CLERK Alpha Tower Suffolk Street Queensway Birmingham B1 1TT (GB)**

(56) References cited:
**IEEE TRANSACTIONS ON INSTRUMENTATION AND MEASUREMENT, vol. 28, no. 4, December 1979, pages 272-278, IEEE, New York, US; C. AKYEL et al.: "Wide-range dynamic complex dielectric constant measurements using microprocessor control techniques"**
**Proceedings of the South African Sugar Technologist's Association - June 1986, Pages 98-100**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Description

This invention relates to an apparatus for controlling a vacuum pan.

In the past, the control of vacuum pan boiling has always been made difficult by the fact that no simple means existed for the direct measurement of supersaturation and crystal content in massecuites. These are the two prime variables to be controlled and, until they can be measured, it is not possible to achieve optimal control of vacuum pan boiling. In the past, measurement of conductivity, viscosity, elevation of boiling point, density and dielectric constant have been used with various degrees of success to control vacuum pans.

In raw sugar factories, electrical conductivity is the most common of these parameters used for pan control. The measurement, however, suffers from a few serious drawbacks:

(1) The conductivity measurement becomes less useful with increasing purity of the boiling massecuite, particularly when the purity is greater than 85.

(2) The conductivity measurement depends on crystal content and mother liquor purity and brix of the massecuite and it is possible to have two different qualities of massecuite having the same conductivity.

(3) When used in continous vacuum pans, conductivity probes are subject to extensive scaling, which has a considerable effect on the reading.

In an article in the International Sugar Journal, Volume 86, pages 73 to 79, there is described an apparatus which measures the dielectric constant of the massecuite and uses this for pan control. However, the dielectric constant varies with both crystal content and the brix of the mother liquor and so measuring the dielectric constant will not provide enough information to know the exact condition of the massecuite.

It is an object of the invention to provide a new apparatus for controlling a vacuum pan in order to obtain a more exact control of the conditions in the massecuite.

According to this invention, there is provided an apparatus for controlling the supersaturation of a mother liquor and the crystal content of massecuite in a vacuum pan, said apparatus comprising a probe for measuring the impedance of the massecuite and located in the vacuum pan at a zone of vigorous circulation, a tuning circuit connected in series with the probe, the probe and the tuning circuit forming part of a voltage divider circuit or a bridge circuit, a signal generator for supplying a radio frequency signal to the voltage divider circuit or the bridge circuit, a detector responsive to an output of the voltage divider circuit or the bridge circuit, and a control device responsive to the output of the detector and providing an output for controlling conditions of the massecuite in the vacuum pan, characterised in that the control device provides an additional output either to the tuning circuit for adjusting an element thereof or to the signal generator for varying the frequency of said radio frequency signal so as to maintain the probe and tuning circuit in resonance, said control device being adapted to compute the resistance and capacitance of the massecuite from the values of the output from the detector and the input to the tuning circuit or the signal generator, and to use the computed values of the resistance and capacitance to provide said output for controlling conditions in the vacuum pan.

The tuning circuit may comprise a series tuned capacitor and inductor network.

Conveniently, the signal generator comprises a radio frequency oscillator, a buffer amplifier for amplifying the output of the oscillator, and a detector and automatic gain control circuit for controlling the output of the buffer amplifier.

Conveniently, a second detector and amplifier circuit may be used to measure the output from the voltage divider or bridge circuit and this may be converted to a 4 to 20 milliamp signal.

In order to calibrate the amplifier and detector circuit, two fixed resistors are switched into the bridge circuit or voltage divider circuit in place of the probe and tuning circuit and gain and offset of the amplifier is adjusted to set span and zero of the measurement circuit. This system is then used to measure the combined impedance of the probe, massecuite and the tuning circuit. By adjusting a variable inductor or capacitor in the tuning circuit, the circuit reactance can be tuned out. At this point, the probe and the tuning circuit are in resonance at the measurement frequency.

There are two alternatives as to how the probe may be used in conjunction with the series-tuned circuit.

(1) There may be provided a means for automatically varying the tuning of the circuit. For example, a varicap diode may be included in the tuning circuit. The control device may take the form of a microprocessor, which is programmed to provide an input signal to the tuning circuit in order to continuously vary the tuning of the probe.

The microprocessor may be programmed to continuously adjust the probe to resonance, measure the output signal from the probe at this point and the signal fed to the tuning circuit and from these measurements together with an experimentally determined value of inductance of the measurement path through the massecuite compute the massecuite parallel resistance and capacitance. These two parameters can then be used for pan control in various ways. In effect, having two outputs means that a massecuite can be fixed in quality and various control philosophies can be derived using these two parameters or various combinations thereof to control a vacuum pan.

(2) The apparatus may be designed such that, instead of a stable oscillator (for example a crystal oscillator) in the signal generator, this is replaced by a variable frequency oscillator. In this case, the tuning circuit may be fixed and the variable frequency oscillator may be controlled by the microprocessor. The microprocessor may be programmed to continuously adjust the probe to resonance as before. By varying the frequency of the oscillator and, from a measurement of probe output at resonance and the frequency of the oscillator the massecuite parallel capacitance and resistance may be calculated as before.

The fact that massecuite capacitance or dielectric constant and resistance can be measured independently, means that this apparatus can be used for controlling high or low grade pans.

The probe can be insulated or non-insulated from the massecuite. In the case of a probe being insulated, calibration is very difficult and, therefore, a non-insulated probe is preferred. Calibration and setting-up of the probe is carried out by connecting resistor capacitor combinations across the probe. An additional inductor and variable capacitors in the probe-tuning circuit allows standardisation of individual probe calibration.

Apart from its other advantages, measurements obtained from the present invention like many other RF probes are less affected by the effects of fouling than conventional conductivity electrodes. Usually fouling of the probe results in inaccurate measurement after a short period of time in massecuites of high purity, but with the present arrangement, it is only when severe fouling takes place that the outputs may be significantly displaced. This makes this type of probe particularly suitable for use in continuous pans. Typically, a probe may operate for periods of one week without the necessity for cleaning in a high grade continuous pan.

An embodiment of the invention is illustrated in the accompanying circuit diagram in which a probe 10 is connected to a tuning circuit 12, whose output passes through a detector and amplifier 14 to a microprocessor controller 16 having outputs 18 and 20 for adjusting the probe to resonance and to control pan parameters in any desired manner.

The radio frequency generator circuit generally includes an RF oscillator 32, and a detector and AGC 34 both connected to a buffer amplifier 36 to control the output voltage fed to resistor R. Resistor R, the probe and tuning circuit constitutes the voltage divider network which could alternatively be replaced by a bridge type network.

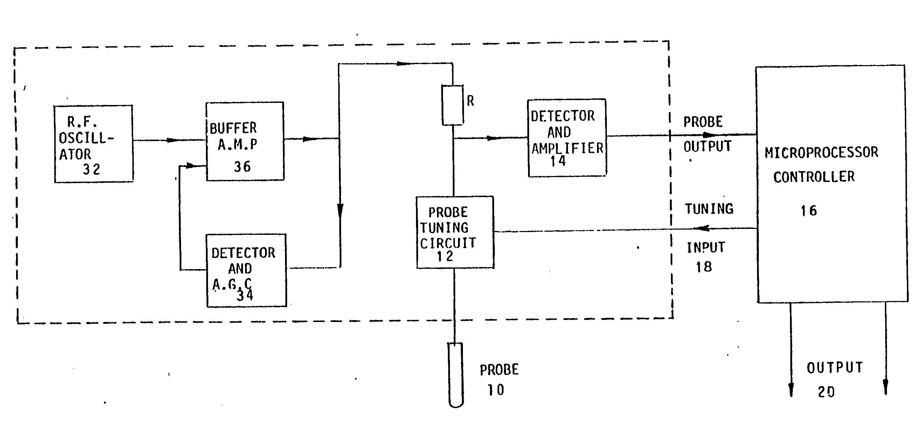

## Claims

1. An apparatus for controlling the supersaturation of a mother liquor and the crystal content of massecuite in a vacuum pan, said apparatus comprising a probe (10) for measuring the impedance of the massecuite and located in the vacuum pan at a zone of vigorous circulation, a tuning circuit (12) connected in series with the probe (10), the probe (10) and the tuning circuit (12) forming part of a voltage divider circuit (10, 12, R) or a bridge circuit, a signal generator (32, 34, 36) for supplying a radio frequency signal to the voltage divider circuit or the bridge circuit, a detector (14) responsive to an output of the voltage divider circuit or the bridge circuit, and a control device (16) responsive to the output of the detector (14) and providing an output (20) for controlling conditions of the massecuite in the vacuum pan, characterised in that the control device (16) provides an additional output (18) either to the tuning circuit (12) for adjusting an element thereof or to the signal generator (32, 34, 36) for varying the frequency of said radio frequency signal so as to maintain the probe (10) and tuning circuit (12) in resonance, said control device (16) being adapted to compute the resistance and capacitance of the massecuite from the values of the output from the detector (14) and the input (18) to the tuning circuit (12) or the signal generator (32, 34, 36), and to use the computed values of the resistance and capacitance to provide said output (20) for controlling conditions in the vacuum pan.

2. An apparatus as claimed in claim 1, characterized in that the signal generator comprises a radio frequency oscillator (32), a buffer amplifier (36) for amplifying the output of the oscillator (32), and a detector and automatic gain control circuit (34) for controlling output of the buffer amplifier (36).

3. An apparatus as claimed in claim 1 or claim 2, characterized in that the detector (14) forms part of a combined detector and amplifier circuit.

## Patentansprüche

1. Vorrichtung zur Steuerung der Übersättigung einer Mutterlauge und des Kristallgehaltes einer Füllmasse in einem Vakuumtrog, mit einer Sonde (10) zur Messung der Impedanz der Füllmasse, die sich im Vakuumtrog in einer Zone heftiger Umwälzung befindet, einem Abstimmkreis (12), der in Reihe zur Sonde (10) liegt, wobei die Sonde (10) und der Abstimmkreis (12) einen Teil einer Spannungsteilerschaltung (10, 12, R) oder einer Brückenschaltung bilden, einem Signalgenerator (32, 34, 36) zur Zuführung eines Hochfrequenzsignals zur Spannungsteilerschaltung oder zur Brückenschaltung, einem Detektor (14), der auf ein Ausgangssignal der Spannungsteilerschaltung oder der Brückenschaltung anspricht, und einer Steuervorrichtung (16), die auf das Ausgangssignal des Detektors (14) anspricht und ein Ausgangssignal (20) zur Steuerung des Zustandes der Füllmasse in Vakuumtrog liefert, dadurch gekennzeichnet, dass die Steuervorrichtung (16) ein zusätzliches Ausgangssignal (18) entweder an die Abstimmschaltung (12) zur Einstellung eines Elementes derselben liefert, oder zum Signalgenerator (32, 34, 36) zur Änderung der Frequenz des Hochfrequenzsignals, um die Sonde (10) und den Abstimmkreis (12) in Resonanz zu halten, dass die Steuervorrichtung (16) in der Lage ist, den Widerstand und die Kapazität der Füllmasse aus den Werten des Ausgangssignals vom Detektor (14) und des Eingangssignals (18) zum Abstimmkreis (12) oder zum Signalgenerator (32, 34, 36) zu berechnen, und die berechneten Widerstands- und Kapazitätswerte zu verwenden, um das Ausgangssignal (20) zur Steuerung des Zustandes im Vakuumtrog zu liefern.

2. Vorrichtung nach Anspruch 1, dadurch gekennzeichnet, dass der Signalgenerator einen Hochfrequenzoszillator (32), einen Trennverstärker (36) zur Verstärkung des Ausgangssignals des Oszillators (32) und einen Detektor und eine automatische Verstärkungsregelungsschaltung (34) zur Steuerung des Ausgangssignals des Trennverstärkers (36) aufweist.

3. Vorrichtung nach Anspruch 1 oder 2, dadurch gekennzeichnet, dass der Detektor (14) einen Teil einer kombinierten Detektor- und Verstärkerschaltung bildet.

**Revendications**

1. Appareil pour contrôler la sursaturation d'une liqueur-mère et de la teneur en cristaux d'une masse cuite dans une chaudière de cuisson, cet appareil comportant une sonde (10) pour mesurer l'impédance de la masse cuite, disposée dans la chaudière de cuisson dans une zone de forte circulatation, un circut d'accord (12) monté en série avec la sonde (10), la sonde (10) et le circuit d'accord (12) faisant partie d'un circuit diviseur de tension (10, 12, R) ou d'un circuit en pont, un générateur de signaux (32, 34, 36) pour fournir un signal haute fréquence au circuit diviseur de tension ou au circuit en pont, un détecteur (14) sensible à une sortie du circuit diviseur de fréquence ou du circuit en pont, et un dispositif de commande (16) sensible à la sortie du détecteur (14) et fournissant une sortie (20) pour régler les conditions de la masse cuite dans la chaudière de cuisson, caractérisé en ce que le dispositif de commande (16) envoie une sortie additionnelle (18), soit au circuit d'accord (12) pour régler un élément de celui-ci, soit au générateur de signaux (32, 34, 36) pour faire varier la fréquence du signal haute fréquence de façon à maintenir en résonance la sonde (10) et le circuit d'accord (12), ce dispositif de commande (16) étant adapté pour calculer la résistance et la capacitance de la masse cuite à partir des valeurs de la sortie du détecteur (14) et de l'entrée (18) au circuit d'accord (12) ou au générateur de signaux (32, 34, 36), et pour utiliser les valeurs calculées de la résistance et de la capacitance pour fournir cette sortie (20) afin de régler les conditions dans la chaudière de cuisson.

2. Appareil selon la revendication 1, caractérisé en ce que le générateur de signaux comporte un oscillateur haute fréquence (32), un amplificateur tampon (36) pour amplifier la sortie de l'oscillateur (32), et un circuit de détection et de commande automatique du gain (34) pour commander la sortie de l'amplificateur tampon (36).

3. Appareil selon la revendication 1 ou la revendication 2, caractérisé en ce que le détecteur (14) fait partie d'un circuit combiné de détection et d'amplification.

MICROPROCESSOR CONTROLLER 16

OUTPUT 20

PROBE OUTPUT

TUNING

INPUT 18

DETECTOR AND AMPLIFIER 14

R

PROBE TUNING CIRCUIT 12

PROBE 10

BUFFER A.M.P 36

DETECTOR AND A.G.C 34

R.F. OSCILLATOR 32